# EUROPÄISCHE PATENTSCHRIFT

(11) **EP 3 404 447 B1**
(45) Veröffentlichungstag und Bekanntmachung des Hinweises auf die Patenterteilung: **14.08.2019**
(21) Anmeldenummer: 17171522.0
(22) Anmeldetag: 17.05.2017
(51) Int. Cl.: G01T 1/24

(54) **RÖNTGENDETEKTOR AUFWEISEND EINE LICHTQUELLE AM TRÄGERELEMENT**
X-RAY DETECTOR COMPRISING A LIGHT SOURCE ON THE HOLDER ELEMENT
DÉTECTEUR À RAYONS X PRÉSENTANT UNE SOURCE LUMINEUSE SUR UN ÉLÉMENT SUPPORT

(43) Veröffentlichungstag der Anmeldung: 21.11.2018
(73) Patentinhaber: Siemens Healthcare GmbH, 91052 Erlangen (DE)
(72) Erfinder: Eismann, Alfons, 91361 Pinzberg (DE); Wölfel, Stefan, 91077 Dormitz (DE)

(56) Entgegenhaltungen:
- DE-A1-102013 214 684
- US-A1- 2007 131 867
- US-A1- 2014 284 456

## Beschreibung

Die Erfindung betrifft einen Röntgendetektor und ein medizinisches Gerät dazu, wobei der Röntgendetektor eine am Trägerelement angeordnete Lichtquelle aufweist, welche ein Konverterelement des Röntgendetektors über einen Lichtpfad beleuchtet.

In der Röntgenbildgebung, beispielsweise in der Computertomographie, der Angiographie oder der Radiographie, können zählende direkt-konvertierende Röntgendetektoren oder integrierende indirekt-konvertierende Röntgendetektoren verwendet werden.

Die Röntgenstrahlung oder die Photonen können in indirektkonvertierenden Röntgendetektoren durch ein geeignetes Konvertermaterial in Licht und mittels Photodioden in elektrische Pulse umgewandelt werden. Als Konvertermaterial werden häufig Szintillatoren, beispielsweise GOS (Gd2O2S), CsI, YGO oder LuTAG, eingesetzt. Szintillatoren werden insbesondere in der medizinischen Röntgenbildgebung im Energiebereich bis 1MeV eingesetzt. Üblicherweise werden sogenannte indirekt-konvertierende Röntgendetektoren, sogenannte Szintillatordetektoren, verwendet, bei denen die Konvertierung der Röntgen- oder Gammastrahlen in elektrische Signale in zwei Stufen erfolgt. In einer ersten Stufe werden die Röntgen- oder Gammaquanten in einem Szintillatorelement absorbiert und in optisch sichtbares Licht umgewandelt, dieser Effekt wird Lumineszenz genannt. Das durch Lumineszenz angeregte Licht wird anschließend in einer zweiten Stufe durch eine mit dem Szintillatorelement optisch gekoppelten ersten Photodiode in ein elektrisches Signal umgewandelt, über eine Auswerte- oder Ausleseelektronik ausgelesen und anschließend an eine Recheneinheit weitergeleitet.

Die Röntgenstrahlung oder die Photonen können in direkt-konvertierenden Röntgendetektoren durch ein geeignetes Konvertermaterial in elektrische Pulse umgewandelt werden. Ein Konverterelement weist das Konvertermaterial auf. Als Konvertermaterial können beispielsweise CdTe, CZT, CdZnTeSe, CdTeSe, CdMnTe, InP, TlBr2, HgI2, GaAs oder andere verwendet werden. An das Konverterelement wird eine Spannung angelegt, so dass die durch die Röntgenstrahlung bzw. Photonen ausgelösten Elektronen-Loch-Paaren getrennt werden können. Die elektrische Ladung der Elektronen oder Löcher wird als elektrischer Puls an die Auswerteelektronik weitergeleitet. Die elektrischen Pulse werden von einer Auswerteelektronik, beispielsweise einem integrierten Schaltkreis (Application Specific Integrated Circuit, ASIC), bewertet. In zählenden Röntgendetektoren wird einfallende Röntgenstrahlung durch Zählen der elektrischen Pulse, welche durch die Absorption von Röntgenphotonen im Konvertermaterial ausgelöst werden, gemessen. Die Höhe des elektrischen Pulses ist in der Regel proportional zur Energie des absorbierten Röntgenphotons. Dadurch kann eine spektrale Information durch den Vergleich der Höhe des elektrischen Pulses mit einem Schwellwert extrahiert werden.

Das Konvertermaterial kann Drifteigenschaften aufweisen, welche sich als Drifteffekt bzw. Veränderung in den Zählwerten der Auswerteelektronik auswirken und somit unerwünschte Bildartefakte erzeugen können. Mittels zusätzlicher Beleuchtung können diese Drifteigenschaften verbessert werden. Durch die zusätzliche Beleuchtung kann das Konvertermaterial beispielsweise konditioniert, d.h. ein vorbestimmter Zustand hergestellt, werden. Durch diese Konditionierung können Störstellen, welche sich im Konvertermaterial befinden, entsprechend reduziert werden.

Aus der Druckschrift DE 10 2010 015 422 A1 ist ein Röntgendetektor bekannt, welcher eine direkt konvertierende Halbleiterschicht zur Wandlung einer eintreffenden Strahlung in elektrische Signale mit einer für die Halbleiterschicht charakteristischen Bandlückenenergie und zumindest eine Lichtquelle zur Einkopplung von Licht in die Halbleiterschicht umfasst, wobei das erzeugte Licht zur Simulation eintreffender Röntgenquanten eine Energie oberhalb der Bandlückenenergie der Halbleiterschicht aufweist. Er umfasst ferner zumindest eine Auswerteeinheit zur Berechnung eines Auswertesignals aus den bei Einkopplung des Lichts in die Halbleiterschicht erzeugten elektrischen Signalen und zumindest eine Kalibriereinheit zur Kalibrierung zumindest eines Pulsdiskriminators auf Basis des Auswertesignals. Hierdurch werden die Voraussetzungen für eine schnell wiederholbare Kalibrierung des Röntgendetektors unter Berücksichtigung des aktuellen Polarisationszustandes ohne Einsatz von Röntgenstrahlung geschaffen.

Aus der Druckschrift DE 10 2011 080 892 B ist ein direktkonvertierender Röntgenstrahlungsdetektor, insbesondere zur Verwendung in CT-Systemen, bekannt, welcher zumindest ein zur Detektion von Röntgenstrahlung verwendetes Halbleitermaterial aufweist. Auf zumindest einer der Röntgenstrahlung zugewandten Seite des Halbleitermaterials ist eine Szintillationsschicht aufgebracht, wobei die Röntgenstrahlung in der Szintillationsschicht optische Strahlung erzeugt.

Aus der Druckschrift DE 10 2013 214 684 A1 ist ein direktkonvertierender Röntgendetektor zur Detektion von Röntgenstrahlung bekannt, welcher einen zur Detektion der Röntgenstrahlung verwendeten Direktkonverter, mindestens einen zumindest teilweise in Strahlungsrichtung der Röntgenstrahlung vor dem Direktkonverter angeordneten Kollimator und mindestens eine Strahlungsquelle aufweist, welche seitlich des Direktkonverters angeordnet ist und den Direktkonverter mit einer zusätzlichen Strahlung indirekt bestrahlt, wobei der mindestens eine Kollimator auf einer dem Direktkonverter zugewandten Seite mindestens eine Reflexionsschicht aufweist, an welcher die zusätzliche Strahlung auf den Direktkonverter reflektiert wird, und aufweisend eine Kühlvorrichtung, durch welche die mindestens eine Strahlungsquelle kühlbar ist.

In der Druckschrift US 2014/284456 A1 wird ein Strahlungsdetektor beschrieben, welcher ein Substrat, einen Pixelbereich auf dem Substrat, welcher ein oder mehrere Pixel mit einem Sensorelement aufweist, und eine Lichtquelle umfasst, wobei die Lichtquelle außerhalb des Pixelbereichs auf einer Seite des Substrats angeordnet ist, die den Pixelbereich aufweist. Dabei wird Licht an einer rückseitigen Oberfläche des Substrats reflektiert und zu einer dem Substrat zugewandten Oberfläche des photoelektrischen Konverterelements eines Sensorelements geleitet.

Die Strahlungsquelle bzw. Lichtquelle wurde bisher beispielsweise auf Leiterplattenmaterial aufgelötet, welche am Kollimator bzw. Streustrahlengitter mittels Klemmung oder Verklebung seitlich zwischen der dem Konverterelement zugewandten Seite des Streustrahlengitters und der Ebene der dem Streustrahlengitter zugewandten Fläche des Konverterelements angebracht wurden.

Die Erfinder haben erkannt, dass durch diese Konstruktion bzw. Anordnung die Wärmeabfuhr der in der Lichtquelle entstehenden Wärme nicht ausreichend sein kann. Die Größe bzw. das Volumen der Leiterplatte kann nur sehr begrenzt sein, da der Strahlengang der Röntgenstrahlung zwischen Streustrahlengitter und Konverterelement nicht von der Leiterplatte beeinflusst werden sollte, um eine nicht-bildwirksame Dosisdeposition im Patienten zu vermeiden. Zudem haben die Erfinder erkannt, dass die Wärmeleitfähigkeit von Leiterplatten in der Regel zu gering ist, so dass die Ableitung der Wärme über das Streustrahlengitter unzureichend ist. Zudem kann die thermische Anbindung des Streustrahlengitters an einen großen Wärmespeicher bzw. Kühlkörper oder an einen vom Streustrahlengitter entfernten Bereich, in dem eine effektive Kühlung, beispielsweise durch (Kühl-)Luft erzielt werden könnte, unzureichend sein.

Der Erfindung liegt das Problem zugrunde, dass durch die unzureichende Wärmeabführung bzw. unzureichende Kühlung die Lichtmenge limitiert ist, da die Lichtquelle bei der Abstrahlung größerer Lichtmengen zu warm werden kann und der sogenannte SOA- Bereich (engl.: Safe-Operation-Area) überschritten werden kann, wodurch die Lebensdauer der Lichtquelle, beispielsweise LEDs, und somit die Lebensdauer des Röntgendetektors einschränkt wird.

Durch diese Limitierung der Lichtmenge kann somit nicht genügend Lichtleistung an das Konvertermaterial gebracht werden, um dies noch besser zu konditionieren. Die negativen Drifteigenschaften können nur unzureichend reduziert werden und eine dauerhaft gute Bildqualität kann im Betrieb kaum gewährleistet werden, sodass die Zählratendrift der Auswerteeinheit zu entsprechend negativen Bildartefakten führen kann.

Es ist Aufgabe der Erfindung, einen Röntgendetektor und ein medizinisches Gerät anzugeben, welche eine verbesserte Kühlung der Lichtquelle ermöglichen, um die Signalstabilität des Röntgendetektors zu verbessern.

Die Aufgabe wird erfindungsgemäß gelöst durch einen Röntgendetektor nach Anspruch 1 und ein medizinisches Gerät nach Anspruch 14.

Die Erfindung betrifft einen Röntgendetektor aufweisend ein Konverterelement und ein Trägerelement in einer Stapelanordnung, wobei eine Lichtquelle am Trägerelement angeordnet ist, und eine Lichtlenkeinheit außerhalb einer Projektion der flächigen Erstreckung des Konverterelements in Stapelrichtung ausgebildet ist. Die Lichtlenkeinheit ist derart angeordnet, dass eine von der Lichtquelle ausgesendete Lichtmenge über einen Lichtpfad auf eine dem Trägerelement abgewandte Oberfläche des Konverterelements einfällt.

Der Röntgendetektor kann insbesondere ein direkt-konvertierender oder zählender Röntgendetektor sein. Das Konverterelement kann insbesondere CdTe oder CZT aufweisen. Der Röntgendetektor kann eine Vielzahl von Pixelelementen aufweisen. Das Konverterelement kann mittels Lötverbindungen mit einer Auswerteeinheit elektrisch leitend verbunden sein. Die Auswerteeinheit kann die Auswerteelektronik umfassen. Die Auswerteeinheit kann, insbesondere elektrisch leitend, mit dem Trägerelement verbunden sein.

Das Trägerelement kann Auswerteelemente, beispielsweise zur Zusammenführung oder Trennung von Datenströmen, aufweisen. Das Trägerelement kann insbesondere mehreren Konverterelementen oder mehreren Auswerteeinheiten zugeordnet sein. Das Trägerelement kann eine Trägerkeramik sein. Die Auswerteeinheit kann, insbesondere elektrisch leitend, direkt mit dem Trägerelement bzw. der Trägerkeramik verbunden sein. Das Trägerelement kann eine sogenannte Modulbackplane sein. Die Auswerteeinheit kann, insbesondere elektrisch leitend, indirekt über ein weiteres Trägerelement mit dem Trägerelement bzw. der Modulbackplane verbunden sein.

Die Stapelanordnung kann die folgenden Elemente in der folgenden Reihenfolge aufweisen: etwaiges Streustrahlengitter, etwaiger Lichtdiffusor, Konverterelement, etwaige Auswerteeinheit, etwaiges weiteres Trägerelement und Trägerelement.

Insbesondere das Konverterelement weist eine flächige Erstreckung senkrecht zur Strahleneinfallsrichtung der Röntgenstrahlung auf. Die flächige Erstreckung ist im Wesentlichen in der Stapelebene ausgebildet. Die durch die flächige Erstreckung ausgebildete Fläche kann als Strahleneinfallsfläche bezeichnet werden. Das Konverterelement weist eine Flächennormale auf, welche senkrecht zur flächigen Erstreckung ausgerichtet ist. Die Stapelrichtung verläuft im Wesentlichen parallel zur Flächennormale. Die Projektion der flächigen Erstreckung des Konverterelements verläuft derart in Stapelrichtung, dass die flächige Erstreckung des Konverterelements bei einer Blickrichtung entlang der Stapelrichtung in eine andere Ebene projiziert wird.

Die Lichtquelle sendet eine, insbesondere vorbestimmte oder/und einstellbare, Lichtmenge aus. Die Lichtquelle ist derart mit einer Lichtlenkeinheit verbunden, so dass die Lichtmenge in die Lichtlenkeinheit im Wesentlichen vollständig eingekoppelt werden kann bzw. die Lichtmenge im Wesentlichen vollständig auf das Konverterelement einfallen kann. Die Lichtmenge wird mittels einer Lichtlenkeinheit entlang eines Lichtpfads zum Konverterelement gelenkt. Der Lichtpfad kann gekrümmt oder geknickt oder nicht-linear ausgebildet sein. Die Lichtmenge fällt auf eine dem Trägerelement abgewandte Oberfläche des Konverterelements ein. Die dem Trägerelement abgewandte Oberfläche kann insbesondere der Lichtquelle abgewandt sein. Die Lichtmenge kann ohne die Lichtlenkeinheit nicht bzw. insbesondere nicht vollständig auf das Konverterelement einfallen.

Die Lichtmenge kann von der Lichtlenkeinheit in den etwaigen Lichtdiffusor im Wesentlichen vollständig eingekoppelt werden. Die Lichtlenkeinheit kann dabei einen kleineren Querschnitt senkrecht zum Lichtpfad aufweisen als der etwaige Lichtdiffusor. Die Lichtlenkeinheit kann nicht als der etwaige Lichtdiffusor ausgebildet sein. Der etwaige Lichtdiffusor kann eine im Wesentlichen homogene Beleuchtung des Konverterelements bewirken. Die Lichtlenkeinheit kann keine im Wesentlichen homogene Beleuchtung des Konverterelements bewirken, insbesondere im Fall eines Lichtleiters. Die Lichtlenkeinheit kann nur zur Umlenkung der Lichtmenge von einem im Wesentlichen zur Stapelrichtung parallelen Verlauf entlang des Lichtpfads hin zu einem im Wesentlichen zur flächigen Erstreckung des Konverterelements parallelen Verlauf entlang des Lichtpfads, beispielsweise hin zum etwaigen Lichtdiffusor, ausgelegt sein. Vorteilhaft kann die Entfernung zwischen der Lichtquelle und dem Konverterelement durch die Anordnung der Lichtquelle am Trägerelement vorteilhaft erhöht werden. Vorteilhaft kann eine Erwärmung des Konverterelements durch die Lichtquelle erheblich reduziert werden. Um eine homogene Konditionierung des flächigen Konverterelements erreichen zu können, ist eine homogene Beleuchtung der Fläche des Konverterelements nötig. Eine Beleuchtung der Seitenflächen parallel zur Stapelrichtung ist nicht ausreichend. Daher schlagen die Erfinder einen Lichtpfad von der Lichtquelle zur dem Trägerelement abgewandten Oberfläche des Konverterelements vor.

Das Trägerelement kann eine HTCC- bzw. LTCC-Keramik aufweisen. Die Lichtquelle ist am Trägerelement angeordnet, beispielsweise mittels einer Lötverbindung. Vorteilhaft kann auf dem Trägerelement mehr Platz für die Lichtquelle bzw. deren Anordnung und Verbindung mit dem Trägerelement vorgesehen sein. Vorteilhaft können die Lötverbindungen größer ausgestaltet werden als es aus der Druckschrift DE 10 2013 214 684 A1 bekannt ist, sodass eine verbesserte Wärmeabführung ermöglicht wird. Die Lichtquelle kann eine LED sein.

Vorteilhaft kann durch die bessere Wärmeabführung der Lichtquelle eine erhöhte Lichtmenge, beispielsweise durch Erhöhung des Stroms in der LED, verwendet werden, wodurch eine erhöhte Lichtleistung auf das Konverterelement einfallen kann. Vorteilhaft kann die Konditionierung des Konverterelements verbessert werden.

Gemäß einem Aspekt der Erfindung ist eine flächige Erstreckung des Trägerelements größer als eine flächige Erstreckung des Konverterelements. Das flächige Konverterelement kann derart gegenüber dem Trägerelement angeordnet sein, dass die Lichtquelle, welche am Trägerelement angeordnet ist, außerhalb der Projektion der flächigen Erstreckung des Konverterelements in Stapelrichtung und auf dem Trägerelement angeordnet ist. Vorteilhaft kann die Lichtquelle seitlich neben der Auswerteeinheit auf dem Trägerelement angeordnet sein.

Gemäß einem Aspekt der Erfindung ist die Lichtquelle außerhalb der Projektion des Konverterelements in Stapelrichtung angeordnet. Vorteilhaft kann die Wärme der Lichtquelle vereinfacht abgeführt werden. Insbesondere vorteilhaft kann das Konverterelement nicht von der Wärme der Lichtquelle beeinflusst sein. Vorteilhaft kann der Lenkwinkel der Lichtlenkeinheit limitiert, beispielsweise maximal 180 Grad, bevorzugt in etwa 90 Grad, sein. Insbesondere kann der Lichtpfad, beispielsweise der gesamte Pfad von der Lichtquelle bis zur Oberfläche des Konverterelements, einen Lenkwinkel von im Wesentlichen maximal 180 Grad aufweisen. Vorteilhaft kann die Lichtlenkeinheit wenig Platz benötigen.

Gemäß einem Aspekt der Erfindung verläuft der Lichtpfad teilweise im Wesentlichen parallel zur Stapelrichtung. Die Lichtmenge kann entlang des Lichtpfads von der Ebene des Trägerelements mindestens zur Ebene des Konverterelements im Wesentlichen entlang der Stapelrichtung geleitet werden. Vorteilhaft kann der Lichtpfad platzsparend ausgebildet sein. Der Lichtpfad kann teilweise im Wesentlichen senkrecht zur Stapelrichtung verlaufen, beispielsweise bis zum seitlichen Rand der Fläche des Konverterelements oder innerhalb eines Lichtdiffusors im Wesentlichen parallel zur flächigen Erstreckung des Konverterelements. Der Lichtdiffusor kann derart ausgebildet sein, dass die Lichtmenge gleichmäßig entlang der flächigen Erstreckung des Konverterelements aus dem Lichtdiffusor auskoppelbar ist, so dass eine homogene Beleuchtung des Konverterelements erreicht werden kann. Der Lichtdiffusor kann den Lichtpfad teilweise umfassen.

Gemäß einem Aspekt der Erfindung umfasst die Lichtlenkeinheit eine Reflexionseinheit. Die Reflexionseinheit kann als Spiegel ausgebildet sein. Die Reflexionseinheit weist zumindest eine reflektierende Fläche auf. Die Reflexionseinheit ist derart ausgebildet, dass der Lichtpfad von der Lichtquelle zur dem Trägerelement abgewandten Oberfläche des Konverterelements verläuft. Die Reflexionseinheit kann am Streustrahlengitter angeordnet sein. Die Reflexionseinheit kann derart am Streustrahlengitter angeordnet sein, dass der Lichtpfad von der Lichtquelle zur Reflexionseinheit parallel zur Stapelrichtung verläuft. Die Reflexionseinheit ist derart ausgebildet, dass der Lichtpfad zur dem Trägerelement abgewandten Oberfläche des Konverterelements oder hin zu einem im Wesentlichen zur flächigen Erstreckung des Konverterelements parallelen Verlauf umgelenkt wird. Die Reflexionseinheit kann zwischen der Befestigungsstruktur des Streustrahlengitters zur Ausrichtung gegenüber dem Stapelaufbau und der dem Konverterelement zugewandten Seite des Streustrahlengitters angeordnet sein, so dass die Wirkung des Streustrahlengitters vorteilhaft nicht durch die Reflexionseinheit bzw. die Lichtlenkeinheit negativ beeinflusst wird.

Vorteilhaft kann die Konditionierung des Konvertermaterials verbessert werden. Vorteilhaft können die negativen Drifteigenschaften des Konvertermaterials und damit die Zählratendrift der Auswerteeinheit verringert werden sowie unerwünschte Bildartefakte reduziert werden. Vorteilhaft kann eine stabile und dauerhaft gute Bildqualität im Betrieb erreicht werden.

Gemäß einem Aspekt der Erfindung umfasst die Lichtlenkeinheit einen Lichtleiter. Der Lichtleiter kann gebogen sein. Der Lichtleiter kann beispielsweise eine Glasfaser umfassen. Vorteilhaft kann eine Anordnung der Lichtlenkeinheit am Streustrahlengitter vermieden werden. Vorteilhaft kann die Lichtlenkeinheit mit dem etwaigen Diffusor lichtleitend verbunden werden. Vorteilhaft kann der Lichtpfad gekrümmt sein. Vorteilhaft kann eine aufwändige Ausrichtung der Reflexionseinheit vermieden werden. Vorteilhaft können Verluste der Lichtmenge entlang des Lichtpfads reduziert werden. Vorteilhaft kann die Lichtmenge direkt an der Lichtquelle in den Lichtleiter eingekoppelt werden. Vorteilhaft kann die etwaige Auswerteeinheit und die Seitenflächen des Konverterelements vor unerwünschtem Lichteinfall geschützt werden.

Gemäß einem Aspekt der Erfindung verläuft der Lichtleiter teilweise durch eine Aussparung eines weiteren Trägerelements. Zwischen dem Konverterelement bzw. der etwaigen Auswerteeinheit und dem Trägerelement kann ein weiteres Trägerelement ausgebildet sein. Das weitere Trägerelement kann eine Trägerkeramik und das Trägerelement die sogenannte Modulbackplane sein. Die Lichtquelle kann an der Modulbackplane angeordnet sein. Vorteilhaft kann die Entfernung der Lichtquelle zum wärmeempfindlichen Konverterelement vergrößert werden. Vorteilhaft kann ein größerer Kühlkörper mit dem Trägerelement thermisch leitend verbunden sein. Der Lichtpfad bzw. das Lichtlenkelement, insbesondere der Lichtleiter, können durch eine Aussparung im weiteren Trägerelement geführt sein. Es kann eine thermische Verbindung der Lichtquelle mittels einer thermischen Durchkontaktierung im Trägerelement zu einem Kühlkörper bzw. einem metallischen Kontakt, beispielsweise einer Haltevorrichtung des Röntgendetektors, ausgebildet sein. Vorteilhaft kann die Wärme der Lichtquelle leichter abgeführt werden.

Gemäß einem Aspekt der Erfindung umfasst das Trägerelement eine thermische Durchkontaktierung. Die thermische Durchkontaktierung kann im Wesentlichen parallel zur Stapelrichtung im Trägerelement ausgebildet sein. Die thermische Durchkontaktierung kann eine thermisch leitende Verbindung zur Lichtquelle aufweisen. Vorteilhaft kann die thermische Leitfähigkeit des Trägerelements verbessert werden. Das Trägerelement kann mittels Wärmeleitkleber vorteilhaft thermisch optimiert mit einer Haltevorrichtung des Röntgendetektors oder einem Kühlkörper bzw. Wärmespeicher/-leiter verbunden sein. Vorteilhaft kann eine Kühlung mittels Kühlluft optimiert werden. Mittels der thermischen Durchkontaktierung kann die Wärmeleitfähigkeit des Trägerelements vorteilhaft verbessert werden, beispielsweise um bis zu einen Faktor 100 bei Anordnung der Lichtquelle auf einem Keramikmaterial gegenüber einer auf FR-4 angeordneten Lichtquelle ohne thermische Durchkontaktierung.

Gemäß einem Aspekt der Erfindung weist das Trägerelement ein elektrisch isolierendes Trägermaterial, einen Verbundwerkstoff oder ein Keramikmaterial auf. Das Keramikmaterial kann bevorzugt HTCC oder LTCC sein. Das Keramikmaterial kann einen Wärmeleitungskoeffizienten von 3 bis 20W/m*K aufweisen. Der Verbundwerkstoff kann FR-4 sein. Der Verbundwerkstoff kann einen Wärmeleitungskoeffizienten von ca. 0.3 W/m*K aufweisen.

Vorteilhaft kann die Kühlung der Lichtquelle optimiert werden.

Gemäß einem Aspekt der Erfindung ist die Lichtlenkeinheit an einer dem Konverterelement zugewandten Oberfläche eines Streustrahlengitters angeordnet. Der Lichtleiter kann entlang der dem Konverterelement zugewandten Oberfläche bzw. Seite des Streustrahlengitters angeordnet sein. Die Reflexionseinheit kann an einer dem Konverterelement zugewandten Oberfläche bzw. Seite des Streustrahlengitters angeordnet sein. Vorteilhaft kann das Streustrahlengitter zur Ausrichtung des Lichtpfads genutzt werden.

Gemäß einem Aspekt der Erfindung weist die Stapelanordnung ferner eine Auswerteeinheit zwischen dem Konverterelement und der Trägerelement auf. Die elektrischen Pulse können direkt in der Auswerteeinheit ausgewertet werden. Vorteilhaft können die Datenströme nah am Konverterelement zusammengefasst werden.

Gemäß einem Aspekt der Erfindung umfasst die Auswerteeinheit oder der Lichtleiter einen für die Lichtmenge intransparenten Lichtschutz. Die Auswerteeinheit kann an den Seitenflächen parallel zur Stapelrichtung einen intransparenten Lichtschutz, beispielsweise eine Beschichtung, aufweisen. Der Lichtleiter kann einen intransparenten Lichtschutz, beispielsweise eine für die Wellenlänge der Lichtquelle intransparente Beschichtung, aufweisen. Vorteilhaft kann die lichtsensible Auswerteeinheit vor Lichteinfall geschützt werden. Vorteilhaft kann eine Beeinflussung der Auswerteeinheit durch den Lichtpfad bzw. die Lichtquelle reduziert bzw. vermieden werden.

Gemäß einem Aspekt der Erfindung sendet die Lichtquelle infrarotes, sichtbares oder ultraviolettes Licht aus. Die Lichtquelle kann als LED ausgebildet sein. Die Lichtquelle kann bevorzugt infrarotes Licht aussenden. Die Wellenlänge der Lichtquelle kann einstellbar sein. Die Lichtmenge bzw. Lichtleistung der Lichtquelle kann einstellbar sein. Vorteilhaft kann die Konditionierung des Konvertermaterials optimiert werden.

Die Erfindung betrifft ferner ein medizinisches Gerät aufweisend einen erfindungsgemäßen Röntgendetektor. Die Vorteile des erfindungsgemäßen Röntgendetektors können auf das medizinische Gerät übertragen werden. Vorteilhaft können Bildartefakte reduziert werden.

Gemäß einem Aspekt der Erfindung ist das medizinische Gerät ein Computertomographiesystem. Vorteilhaft kann die Kühlung bzw. Wärmeabfuhr von der Lichtquelle raumeffizient ausgestaltet sein, so dass innerhalb des Rotors nötiger Platz zur Kühlung der Lichtquelle minimiert werden kann.

Nachfolgend werden Ausführungsbeispiele der Erfindung anhand von Zeichnungen näher erläutert. Hierbei zeigt:
FIG 1 schematisch einen erfindungsgemäßen Röntgendetektor in einer ersten Ausführungsform;
FIG 2 schematisch einen erfindungsgemäßen Röntgendetektor in einer zweiten Ausführungsform;
FIG 3 schematisch einen erfindungsgemäßen Röntgendetektor in einer dritten Ausführungsform;
FIG 4 schematisch einen erfindungsgemäßen Röntgendetektor in einer vierten Ausführungsform; und
FIG 5 schematisch eine Darstellung eines erfindungsgemäßen Computertomographiesystems.

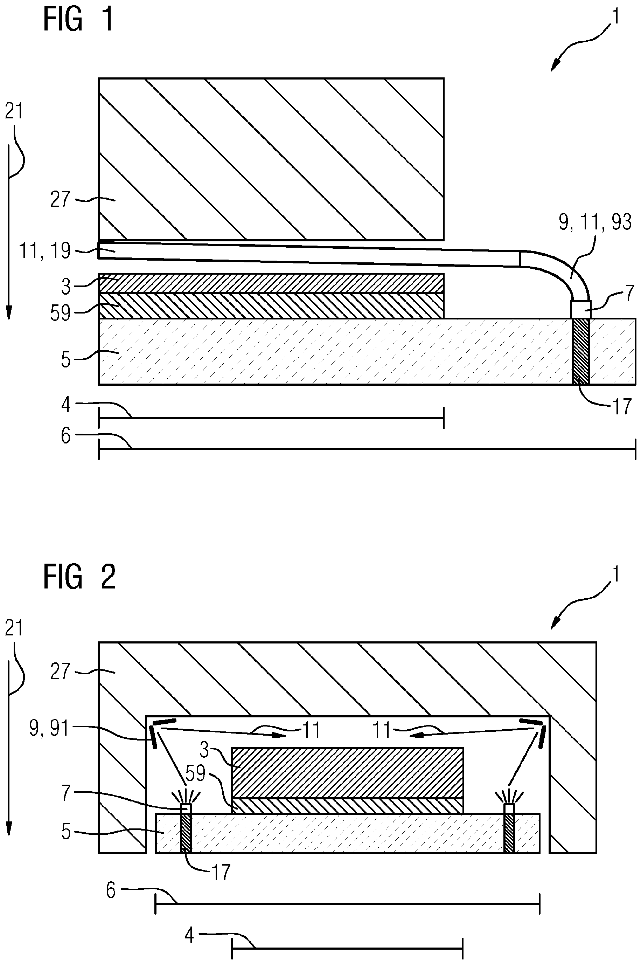

Die Fig. 1 zeigt eine beispielhafte Ausführung des erfindungsgemäßen Röntgendetektors 1 in einer ersten Ausführungsform. Der Röntgendetektor 1 weist ein Konverterelement 3 und ein Trägerelement 5 in einer Stapelanordnung auf. Eine Lichtquelle 7 ist am Trägerelement 5 angeordnet. Eine Lichtlenkeinheit 9, 93 ist außerhalb einer Projektion der flächigen Erstreckung des Konverterelements 3 in Stapelrichtung 21 ausgebildet. Die Lichtlenkeinheit 9, 93 ist derart angeordnet, dass eine von der Lichtquelle 7 ausgesendete Lichtmenge über einen Lichtpfad 11 auf eine dem Trägerelement 5 abgewandte Oberfläche des Konverterelements 3 einfällt. Eine flächige Erstreckung 6 des Trägerelements 5 ist größer als eine flächige Erstreckung 4 des Konverterelements 3. Die Lichtquelle 7 ist außerhalb der Projektion des Konverterelements 3 in Stapelrichtung 21 angeordnet. Die Lichtlenkeinheit 9, 93 umfasst einen Lichtleiter 93. Das Trägerelement 5 umfasst eine thermische Durchkontaktierung 17. Das Trägerelement 5 weist ein elektrisch isolierendes Trägermaterial, einen Verbundwerkstoff oder ein Keramikmaterial auf. Die Stapelanordnung weist ferner eine Auswerteeinheit 59 zwischen dem Konverterelement 3 und der Trägerelement 5 auf. Die Auswerteeinheit 59 oder/und der Lichtleiter 9, 93 umfassen einen für die Lichtmenge intransparenten Lichtschutz. Die Lichtquelle 7 ist eine LED. Die Lichtquelle 7 sendet infrarotes, sichtbares oder ultraviolettes Licht aus. Bevorzugt sendet die Lichtquelle 7 infrarotes Licht aus.

Der Röntgendetektor 1 ist ein direkt-konvertierender oder zählender Röntgendetektor. Das Konverterelement 3 weist CdTe oder CZT auf. Der Röntgendetektor 1 weist eine Vielzahl von Pixelelementen auf. Das Konverterelement 3 ist mittels Lötverbindungen mit einer Auswerteeinheit 59 elektrisch leitend verbunden. Die Auswerteeinheit 59 umfasst die Auswerteelektronik. Die Auswerteeinheit 59 ist elektrisch leitend mit dem Trägerelement 5 verbunden.

Das Trägerelement 5 ist eine Trägerkeramik. Die Auswerteeinheit 59 ist insbesondere elektrisch leitend direkt mit dem Trägerelement 5 bzw. der Trägerkeramik verbunden. Die Stapelanordnung weist die folgenden Elemente in der folgenden Reihenfolge auf: Streustrahlengitter 27, Lichtdiffusor 19, Konverterelement 3, Auswerteeinheit 59 und Trägerelement 5.

Das Konverterelement 3 weist eine flächige Erstreckung 4 senkrecht zur betriebsgemäßen Strahleneinfallsrichtung der Röntgenstrahlung bzw. Stapelrichtung 21 auf. Die flächige Erstreckung 4 ist im Wesentlichen in der Stapelebene ausgebildet. Die durch die flächige Erstreckung 4 ausgebildete Fläche wird als Strahleneinfallsfläche bezeichnet. Das Konverterelement 3 weist eine Flächennormale auf, welche senkrecht zur flächigen Erstreckung 4 ausgerichtet ist. Die Stapelrichtung 21 verläuft im Wesentlichen parallel zur Flächennormale. Die Projektion der flächigen Erstreckung 4 des Konverterelements 3 verläuft derart in Stapelrichtung 21, dass die flächige Erstreckung 4 des Konverterelements 3 bei einer Blickrichtung entlang der Stapelrichtung 21 in eine andere Ebene, insbesondere andere Stapelebene, projiziert wird.

Die Lichtquelle 7 sendet eine, insbesondere vorbestimmte und einstellbare, Lichtmenge aus. Die Lichtquelle 7 ist derart mit einer Lichtlenkeinheit 9, 93 verbunden, so dass die Lichtmenge in die Lichtlenkeinheit 9, 93 im Wesentlichen vollständig eingekoppelt wird bzw. die Lichtmenge im Wesentlichen vollständig auf das Konverterelement 3 einfällt. Die Lichtmenge wird mittels einer Lichtlenkeinheit 9, 93 entlang eines Lichtpfads 11 zum Konverterelement 3 gelenkt. Der Lichtpfad 11 ist gekrümmt oder geknickt oder nicht-linear ausgebildet. Die Lichtmenge fällt auf eine dem Trägerelement 5 abgewandte Oberfläche des Konverterelements 3 ein. Die dem Trägerelement 5 abgewandte Oberfläche des Konverterelements 3 kann insbesondere der Lichtquelle 7 abgewandt sein. Die Lichtmenge kann ohne die Lichtlenkeinheit 9, 93 nicht bzw. insbesondere nicht vollständig auf das Konverterelement 3 einfallen. Das Trägerelement 5 weist eine HTCC- bzw. LTCC-Keramik auf. Die Lichtquelle 7 ist am Trägerelement 5 angeordnet, beispielsweise mittels einer Lötverbindung.

Das flächige Konverterelement 3 ist derart gegenüber dem Trägerelement 5 angeordnet, dass die Lichtquelle 7 außerhalb der Projektion der flächigen Erstreckung 4 des Konverterelements 3 in Stapelrichtung 21 und auf dem Trägerelement 5 angeordnet ist. Die Lichtquelle 7 ist seitlich neben der Auswerteeinheit 59 auf dem Trägerelement 5 angeordnet. Der Lenkwinkel der Lichtlenkeinheit 9, 93 kann beispielsweise in etwa 90 Grad betragen.

Die Lichtmenge wird in der in Fig.1 gezeigten Ausführungsform entlang des Lichtpfads 11 von der Ebene des Trägerelements 5 mindestens zur Ebene des Konverterelements 3 im Wesentlichen entlang der Stapelrichtung 21 geleitet. Der Lichtpfad 11 verläuft dann teilweise im Wesentlichen senkrecht zur Stapelrichtung 21, beispielsweise bis zum seitlichen Rand der Fläche des Konverterelements 3 oder innerhalb eines Lichtdiffusors 19 im Wesentlichen parallel zur flächigen Erstreckung 4 des Konverterelements 3. Der Lichtdiffusor 19 kann derart ausgebildet sein, dass die Lichtmenge gleichmäßig entlang der flächigen Erstreckung 4 des Konverterelements 3 aus dem Lichtdiffusor 19 auskoppelbar ist, so dass eine homogene Beleuchtung des Konverterelements 3 erreicht werden kann. Der Lichtdiffusor 19 kann den Lichtpfad 11 teilweise umfassen. Der Lichtleiter 9, 93 ist gebogen. Der Lichtleiter 9, 93 umfasst beispielsweise eine Glasfaser.

Die thermische Durchkontaktierung 17 ist parallel zur Stapelrichtung 21 im Trägerelement 5 ausgebildet. Die thermische Durchkontaktierung 17 weist eine thermisch leitende Verbindung zur Lichtquelle 7 auf. Das Trägerelement 5 kann mittels Wärmeleitkleber thermisch optimiert mit einer Haltevorrichtung des Röntgendetektors 1 oder einem Kühlkörper bzw. Wärmespeicher/-leiter verbunden sein. Mittels der thermischen Durchkontaktierung 17 wird die Wärmeleitfähigkeit des Trägerelements 5 verbessert, beispielsweise um bis zu einen Faktor 100 bei Anordnung der Lichtquelle 7 auf einem Keramikmaterial gegenüber einer auf FR-4 angeordneten Lichtquelle 7 ohne thermische Durchkontaktierung 17. Das Keramikmaterial ist bevorzugt HTCC oder LTCC. Das Keramikmaterial weist einen Wärmeleitungskoeffizienten von 3 bis 20W/m*K auf.

Die Auswerteeinheit 59 kann an den Seitenflächen parallel zur Stapelrichtung 21 einen intransparenten Lichtschutz, beispielsweise eine Beschichtung, aufweisen. Der Lichtleiter 9, 93 kann einen intransparenten Lichtschutz, beispielsweise eine Beschichtung, aufweisen. Die Wellenlänge der Lichtquelle kann einstellbar sein. Die Lichtmenge bzw. Lichtleistung der Lichtquelle 7 kann einstellbar sein.

Die Fig. 2 zeigt eine beispielhafte Ausführung des erfindungsgemäßen Röntgendetektors 1 in einer zweiten Ausführungsform. Die Lichtlenkeinheit 9 umfasst eine Reflexionseinheit 91. Die Lichtlenkeinheit 9, 91 ist an einer dem Konverterelement 3 zugewandten Oberfläche eines Streustrahlengitters 27 angeordnet. Die Reflexionseinheit 9, 91 ist als Spiegel ausgebildet. Die Reflexionseinheit 9, 91 weist zumindest eine reflektierende Fläche auf. Die Reflexionseinheit 9, 91 ist derart ausgebildet, dass der Lichtpfad 11 von der Lichtquelle 7 zur dem Trägerelement 5 abgewandten Oberfläche des Konverterelements 3 verläuft. Die Reflexionseinheit 9, 91 ist am Streustrahlengitter 27 angeordnet. Die Reflexionseinheit 9, 91 ist derart am Streustrahlengitter 27 angeordnet, dass der Lichtpfad 11 von der Lichtquelle 7 zur Reflexionseinheit 9, 91 parallel zur Stapelrichtung 21 verläuft. Die Reflexionseinheit 9, 91 ist derart ausgebildet, dass der Lichtpfad 11 zur dem Trägerelement 5 abgewandten Oberfläche des Konverterelements 3 umgelenkt wird. Die Reflexionseinheit 9, 91 ist zwischen der Befestigungsstruktur des Streustrahlengitters 27 zur Ausrichtung gegenüber dem Stapelaufbau und der dem Konverterelement 3 zugewandten Seite des Streustrahlengitters 27 angeordnet. Die Reflexionseinheit 9, 91 ist an einer dem Konverterelement zugewandten Oberfläche bzw. Seite des Streustrahlengitters 27 angeordnet.

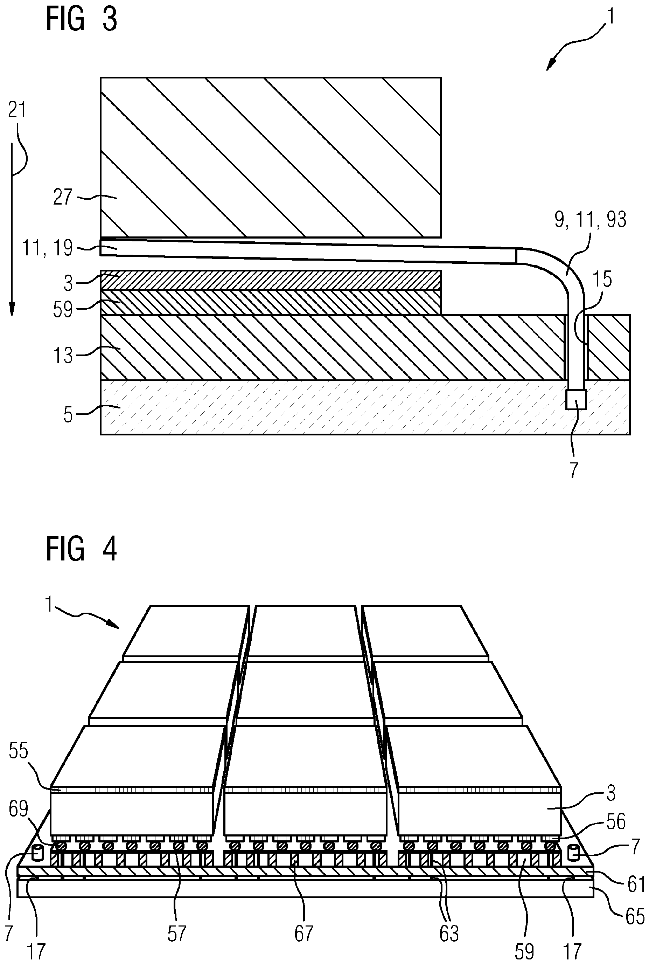

Die Fig. 3 zeigt eine beispielhafte Ausführung des erfindungsgemäßen Röntgendetektors 1 in einer dritten Ausführungsform. Der Lichtleiter 9, 93 verläuft teilweise durch eine Aussparung 15 eines weiteren Trägerelements 13. Der Lichtpfad 11 verläuft teilweise im Wesentlichen parallel zur Stapelrichtung 21. Die Stapelanordnung weist die folgenden Elemente in der folgenden Reihenfolge auf: Streustrahlengitter 27, Lichtdiffusor 19, Konverterelement 3, Auswerteeinheit 59, weiteres Trägerelement 13 und Trägerelement 5.Das Trägerelement 5 ist eine sogenannte Modulbackplane. Die Auswerteeinheit 59 ist elektrisch leitend, indirekt über ein weiteres Trägerelement 13 mit dem Trägerelement 5 bzw. der Modulbackplane verbunden.

Zwischen dem Konverterelement 3 bzw. der Auswerteeinheit 59 und dem Trägerelement 5 ist ein weiteres Trägerelement 13 ausgebildet bzw. angeordnet. Das weitere Trägerelement 13 ist eine Trägerkeramik und das Trägerelement 5 ist die sogenannte Modulbackplane. Die Lichtquelle 7 ist an der Modulbackplane angeordnet. Der Lichtpfad 11 bzw. die Lichtlenkeinheit 9, insbesondere der Lichtleiter 93, ist durch eine Aussparung 15 im weiteren Trägerelement 13 geführt. Es kann eine thermische Verbindung der Lichtquelle 7 mittels einer thermischen Durchkontaktierung im Trägerelement 5 zu einem Kühlkörper bzw. einem metallischen Kontakt, beispielsweise einer Haltevorrichtung des Röntgendetektors 1, ausgebildet sein (nicht dargestellt) .

Die Fig. 4 zeigt eine beispielhafte Ausführung des erfindungsgemäßen Röntgendetektors 1 in einer vierten Ausführungsform mit einer Anordnung von mehreren direkt-konvertierenden Konverterelementen 3 und Auswerteeinheiten 59 in Stapelanordnungen. Jeweils ein direkt-konvertierendes Konverterelement 3 und jeweils eine Auswerteeinheit 59 liegen in einer Stapelanordnung vor. In einer bevorzugten Ausführungsform weist der Röntgendetektor 1 eine zweidimensionale Matrix oder Anordnung einer Mehrzahl von Detektorelementen, Pixeln oder Subpixeln, auf. Die Anzahl der Subpixel kann beispielsweise im Bereich von 100 bis mehrere Tausend liegen. Die Subpixel können mehrere Energiekanäle aufweisen. Das direkt-konvertierende Konverterelement 3 kann als flächenhafter Direktkonverter, beispielsweise aufweisend CdTe, CZT, CdZnTeSe, CdTeSe, CdMnTe, InP, TlBr2, HgI2, GaAs oder andere als Konvertermaterial, ausgebildet sein. Die erste Oberfläche des direkt-konvertierenden Konverterelements 3 weist eine Top-Elektrode 55 auf. Die zweite Oberfläche des direkt-konvertierenden Konverterelements 3 weist eine zweidimensionale Anordnung von Kontakten 56 bzw. Elektroden auf. Die Kontakte 56 sind über Lötverbindungen 69 mit den Pixelelektroden 57 und den Pixelelektroniken 67 in der Auswerteeinheit 59 verbunden. Die einzelnen Pixelelektroden 57 können insbesondere voneinander separiert sein (nicht dargestellt). Die Lötverbindungen 69 können beispielsweise als Lötkugeln (bump bonds oder bump balls) oder Lötmaterial in Verbindung mit Kupfersäulen (copper pillars) ausgebildet sein. Die Anzahl der Kontakte 56, die Anzahl der Lötverbindungen 69, die Anzahl der Pixelelektroden 57 und die Anzahl der Pixelelektroniken 67 in der Auswerteeinheit 59 sind gleich. Das elektrische Feld zwischen der Top-Elektrode 55 und einem Kontakt 56 bestimmt ein sensitives Detektionsvolumen. Die Einheit aus einem Detektionsvolumen, einem Kontakt 56, einer Lötverbindung 69, einer Pixelelektrode 57 und einer mit der Pixelelektrode 57 verbundenen Pixelelektronik 67 bildet ein Detektorelement. Die Auswerteeinheit 59 ist an der Unterseite mit einem Substrat 61 verbunden. Die Lichtquelle 7 ist auf dem Substrat 61 angeordnet. Die Lichtquelle 7 ist in thermisch leitender Verbindung mit einer thermischen Durchkontaktierung 17. Die Auswerteeinheit 59 ist über TSV-Verbindungen 63 durch das Substrat 61 hindurch mit einer peripheren Elektronik 65 verbunden. Zur besseren Übersichtlichkeit sind das Streustrahlengitter, der Lichtpfad und die Lichtlenkeinheit in dieser Ansicht nicht dargestellt.

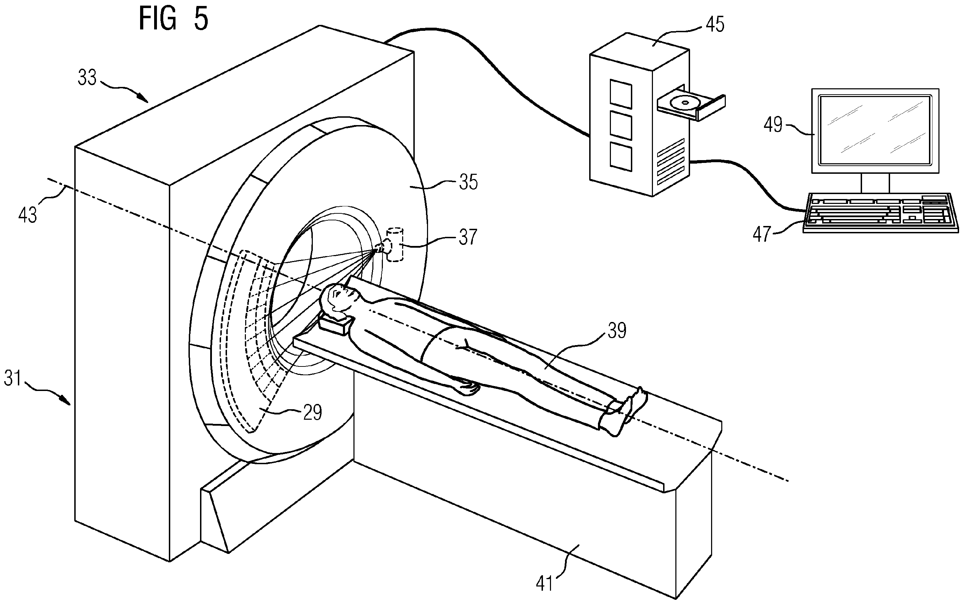

Die Fig. 5 zeigt eine beispielhafte Ausführung eines erfindungsgemäßen Computertomographiesystems 31 mit einem erfindungsgemäßen Röntgendetektor 1. Das Computertomographiesystem 31 beinhaltet eine Gantry 33 mit einem Rotor 35. Der Rotor 35 umfasst eine Röntgenquelle 37 und eine Detektorvorrichtung 29 mit einer Mehrzahl von erfindungsgemäßen Röntgendetektoren. Der Patient 39 ist auf der Patientenliege 41 gelagert und ist entlang der Rotationsachse z 43 durch die Gantry 33 bewegbar. Zur Steuerung und Berechnung der Schnittbilder wird eine Recheneinheit 45 verwendet. Eine Eingabeeinrichtung 47 und eine Ausgabevorrichtung 49 sind mit der Recheneinheit 45 verbunden.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Röntgendetektor (1) aufweisend ein Konverterelement (3) und ein Trägerelement (5) in einer Stapelanordnung, wobei
a. eine Lichtquelle (7) am Trägerelement (5) angeordnet ist,
b. eine Lichtlenkeinheit (9) außerhalb einer Projektion einer flächigen Erstreckung (4) des Konverterelements (3) in Stapelrichtung ausgebildet ist,
**dadurch gekennzeichnet, dass**
die Lichtlenkeinheit (9) derart angeordnet ist, dass eine von der Lichtquelle (7) ausgesendete Lichtmenge über einen Lichtpfad (11) auf eine dem Trägerelement (5) abgewandte Oberfläche des Konverterelements (3) einfällt.

2. Röntgendetektor (1) nach Anspruch 1, wobei eine flächige Erstreckung (6) des Trägerelements (5) größer als eine flächige Erstreckung (4) des Konverterelements (3) ist.

3. Röntgendetektor (1) nach Anspruch 2, wobei die Lichtquelle (7) außerhalb der Projektion des Konverterelements (3) in Stapelrichtung angeordnet ist.

4. Röntgendetektor (1) nach einem der vorangehenden Ansprüche, wobei der Lichtpfad (11) teilweise parallel zur Stapelrichtung verläuft.

5. Röntgendetektor (1) nach einem der vorangehenden Ansprüche, wobei die Lichtlenkeinheit (9) eine Reflexionseinheit (91) umfasst.

6. Röntgendetektor (1) nach einem der Ansprüche 1 bis 4, wobei die Lichtlenkeinheit (9) einen Lichtleiter (93) umfasst.

7. Röntgendetektor (1) nach Anspruch 6, aufweisend ein weiteres Trägerelement (13), wobei der Lichtleiter (93) teilweise durch eine Aussparung (15) des weiteren Trägerelements (13) verläuft.

8. Röntgendetektor (1) nach einem der vorangehenden Ansprüche, wobei das Trägerelement (5) eine thermische Durchkontaktierung (17) umfasst.

9. Röntgendetektor (1) nach einem der vorangehenden Ansprüche, wobei das Trägerelement (5) ein elektrisch isolierendes Trägermaterial, einen Verbundwerkstoff oder ein Keramikmaterial aufweist.

10. Röntgendetektor (1) nach einem der vorangehenden Ansprüche, aufweisend ein Streustrahlengitter (27), wobei die Lichtlenkeinheit (9) an einer dem Konverterelement (3) zugewandten Oberfläche des Streustrahlengitters (27) angeordnet ist.

11. Röntgendetektor (1) nach einem der vorangehenden Ansprüche, wobei die Stapelanordnung ferner eine Auswerteeinheit (59) zwischen dem Konverterelement (3) und der Trägerelement (5) aufweist.

12. Röntgendetektor (1) nach Anspruch 11, wobei die Auswerteeinheit (59) oder der Lichtleiter (9) einen für die Lichtmenge intransparenten Lichtschutz umfasst.

13. Röntgendetektor (1) nach einem der vorangehenden Ansprüche, wobei die Lichtquelle (7) infrarotes, sichtbares oder ultraviolettes Licht aussendet.

14. Medizinisches Gerät aufweisend einen Röntgendetektor (1) nach einem der vorangehenden Ansprüche.

15. Medizinisches Gerät nach Anspruch 14, wobei das medizinische Gerät ein Computertomographiesystem (31) ist.

## Claims

1. X-ray detector (1) having a converter element (3) and a carrier element (5) in a stack arrangement, wherein
a. a light source (7) is arranged on the carrier element (5),
b. a light-directing unit (9) is formed outside a projection of a planar extension (4) of the converter element (3) in the stacking direction,
**characterised in that**
the light-directing unit (9) is arranged such that a quantity of light emitted by the light source (7) is incident on a surface of the converter element (3) facing away from the carrier element (5) by way of a light path (11).

2. X-ray detector (1) according to claim 1, wherein a planar extension (6) of the carrier element (5) is greater than a planar extension (4) of the converter element (3).

3. X-ray detector (1) according to claim 2, wherein the light source (7) is arranged outside the projection of the converter element (3) in the stacking direction.

4. X-ray detector (1) according to one of the preceding claims, wherein the light path (11) partially runs essentially parallel to the stacking direction.

5. X-ray detector (1) according to one of the preceding claims, wherein the light-directing unit (9) comprises a reflection unit (91).

6. X-ray detector (1) according to one of the claims 1 to 4, wherein the light-directing unit (9) comprises a light guide (93).

7. X-ray detector (1) according to claim 6, having a further carrier element (13), wherein the light guide (93) partially runs through a recess (15) of the further carrier element (13).

8. X-ray detector (1) according to one of the preceding claims, wherein the carrier element (5) comprises a thermal through-connection (17).

9. X-ray detector (1) according to one of the preceding claims, wherein the carrier element (5) comprises an electrically insulating carrier material, a composite material or a ceramic material.

10. X-ray detector (1) according to one of the preceding claims, having an anti-scatter grid (27), wherein the light-directing unit (9) is arranged on a surface of the anti-scatter grid (27) facing the converter element (3).

11. X-ray detector (1) according to one of the preceding claims, wherein the stack arrangement also has an evaluation unit (59) between the converter element (3) and the carrier element (5).

12. X-ray detector (1) according to claim 11, wherein the evaluation unit (59) or the light guide (9) comprises a light screen opaque for the quantity of light.

13. X-ray detector (1) according to one of the preceding claims, wherein the light source (7) emits infrared, visible or ultraviolet light.

14. Medical device having an X-ray detector (1) according to one of the preceding claims.

15. Medical device according to claim 14, wherein the medical device is a computed tomography system (31).

## Revendications

1. Détecteur (1) à rayons X, comportant un élément (3) de convertisseur et un élément (5) de support suivant un agencement en empilement, dans lequel
a. une source (7) lumineuse est disposée sur l'élément (5) de support,
b. une unité (9) de déviation de la lumière est constituée à l'extérieur d'une projection d'une étendue (4) plane de l'élément (3) de convertisseur dans la direction d'empilement,
**caractérisé en ce que**
l'unité (9) de déviation est disposée, de manière à ce qu'une quantité de lumière, émise par la source (7) lumineuse, arrive par un trajet (11) de lumière, sur une surface, opposée à l'élément (5) de support, de l'élément (3) de convertisseur.

2. Détecteur (1) à rayons X suivant la revendication 1, dans lequel une étendue (6) plane de l'élément (5) de support est plus grande qu'une étendue (4) plane de l'élément (3) de convertisseur.

3. Détecteur (1) à rayons X suivant la revendication 2, dans lequel la source (7) lumineuse est disposée à l'extérieur de la projection de l'élément (3) de convertisseur dans la direction d'empilement.

4. Détecteur (1) à rayons X suivant l'une des revendications précédentes, dans lequel le trajet (11) de la lumière s'étend en partie parallèlement à la direction d'empilement.

5. Détecteur (1) à rayons X suivant l'une des revendications précédentes, dans lequel l'unité (9) de déviation de la lumière comprend une unité (91) de réflexion.

6. Détecteur (1) à rayons X suivant l'une des revendications 1 à 4, dans lequel l'unité (9) de déviation de la lumière comprend une fibre (93) optique.

7. Détecteur (1) à rayons X suivant la revendication 6, comportant un autre élément (13) de support, la fibre (93) optique s'étendant, en partie, dans un évidement (15) de l'autre élément (13) de support.

8. Détecteur (1) à rayons X suivant l'une des revendications précédentes, dans lequel l'élément (5) de support comprend une traversée (17) thermique.

9. Détecteur (1) à rayons X suivant l'une des revendications précédentes, dans lequel l'élément (5) de support a un matériau de support isolant du point de vue électrique, un matériau composite ou un matériau céramique.

10. Détecteur (1) à rayons X suivant l'une des revendications précédentes, comportant un réseau (27) de diffusion, l'unité (9) de déviation de la lumière étant disposée sur une surface, à l'opposé de l'élément (3) de convertisseur, du réseau (27) de diffusion.

11. Détecteur (1) à rayons X suivant l'une des revendications précédentes, dans lequel l'agencement d'empilement a, en outre, une unité (59) d'exploitation entre l'élément (3) de convertisseur et l'élément (5) de support.

12. Détecteur (1) à rayons X suivant la revendication 11, dans lequel l'unité (59) d'exploitation ou la fibre (9) optique comprend une protection non transparente vis-à-vis de l'énergie lumineuse.

13. Détecteur (1) à rayons X suivant l'une des revendications précédentes, dans lequel la source (7) lumineuse émet de la lumière infrarouge visible ou ultraviolette.

14. Appareil médical, comportant un détecteur (1) à rayons X suivant l'une des revendications précédentes.

15. Appareil médical suivant la revendication 14, dans lequel l'appareil médical est un système (31 ) de tomographie à ordinateur.
